(19) Europäisches Patentamt

European Patent Office

Office européen des brevets

(11) **EP 1 918 382 A1**

(12) **EUROPEAN PATENT APPLICATION**

(43) Date of publication:
**07.05.2008 Bulletin 2008/19**

(51) Int Cl.:
***C12P 13/02*** *(2006.01)*

(21) Application number: **06022821.0**

(22) Date of filing: **02.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **DSM IP Assets B.V.**
**6411 TE  Heerlen (NL)**

(72) Inventor: **Flores-Candia, Juana-Lucia**
**4126 Bettingen (CH)**

(74) Representative: **Keller, Günter et al**
**Lederer & Keller**
**Patentanwälte**
**Prinzregentenstrasse 16**
**80538 München (DE)**

(54) **Semi-continuous cascade fermentation process for pantothenate production**

(57)    The present invention relates to a new semi-continuous cascade system (SCCS) for the production of pantothenate by fermentation. This process provides an improved volumetric productivity and enhanced pantothenate yield. This is achieved by the new fermentation process using the semi-continuous cascade system, which comprises a setup of at least 2 fermentation tanks interconnected in a row. Fermentation is carried out in these tanks applying feeding profiles able to modulate Carbon/Nitrogen/Phosphor (C/N/P) at levels that keeps central metabolism active but allows enough C/N for production. Fermented medium transfers are performed from one tank into the next tank in an attempt to extend its biocatalitic activity for additional reaction time ($\Delta t$) before being harvested. Medium withdrawals are performed from the tanks into a separated tank and subjected to downstream operations. The medium transfers and medium withdrawals are calculated according to the present invention so that the volume in each tank remains within a set target +/- 10%. This invention also discloses that glutamate in the presence of ammonia ions improve the utilization of the N-sources.

Figure 4.

**Description**

The present invention relates to a new semi-continuous cascade system (SCCS) for the production of pantothenate by fermentation. This process provides an improved volumetric productivity and yield.

Pantothenate is also known as pantothenic acid or vitamin B5 and its calcium form as CalPan. This water soluble vitamin is an essential nutrient for mammals, including livestock and humans. In cells, pantothenate is primarily used for the biosynthesis of coenzyme A (CoA) and acyl carrier protein (ACP). These coenzymes function in the metabolism of acyl moieties which form thioesters with the sulfide group of the 4'-phosphopantheine portion of these molecules. These coenzymes are essential in all cells, participating in over 100 different intermediary reactions in cellular metabolism.

The main route for synthesizing pantothenate is via chemical synthesis from bulk chemicals. The drastic price reductions on the market and the excessive substrate costs for the chemical synthesis as well as the requirements for optical resolution of racemic intermediates lead to an interest in an improved fermentation production of pantothenate.

To make CalPan fermentation a convincing commercial alternative, it is important to produce pantothenate at the lowest cost possible. Consequently, high substrate conversion efficiency ($Y_{P/S}$, product yield) and high fermenter throughput ($P_V$, volumetric productivity) are key targets. Different approaches exist to increase the production performance considering that the product yield ($Y_{P/S}$) is primarily regarded as strain specific and that the volumetric productivity ($P_V$) and product concentration (P) are a result of the operation mode.

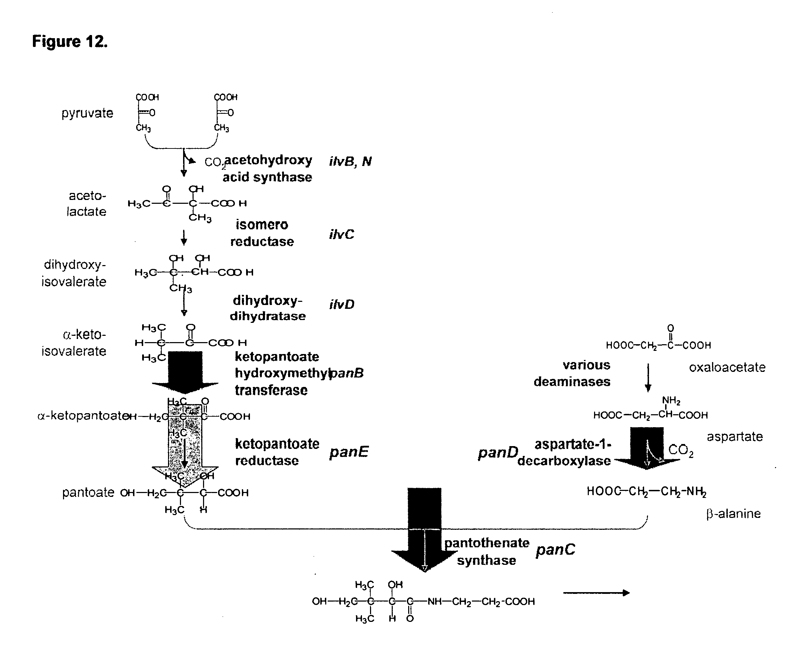

The pantothenate production in bacteria results from the condensation of pantoate and β-alanine and involves the pantothenate biosynthetic enzymes ketopanthoate hydroxylmethyltransferase (the *panB* gene product), ketopanthoate reductase (the *panE* gene product), aspartate-A-decarboxylase (the *panD* gene product) and pantothenate synthetase (the *panC* gene product) as can be seen in Figure 12.

WO 01/21772 discloses a method for increasing the productivity of a *Bacillus* strain by increasing the expression of the *panB, panC, panD* and *panE* genes and by increasing the expression of theilvBNC and ilvD, genes. The resulting strains convert glucose (pyruvate) to commercially attractive quantities of pantothenate.

WO 2004/005525 discloses methods for further enhancing pantothenate production by modulating a biosynthetic pathway that supplies a substrate for the pantothenate biosynthetic pathway, namely the methylene tetrahydrofolate (MTF). In particular, it has been discovered that increasing levels of MTF by modification of the MTF biosynthetic pathway results in enhanced levels of the key pantothenate biosynthetic pathway intermediate, ketopanthoate. Enhanced ketopanthoate levels, in turn, result in significantly enhanced pantothenate production levels in appropriately engineered strains. WO 2004/005525 describes at least three effective means of modifying the MTF biosynthetic pathway.

US 60/262,995 discloses pantothenate production strains that have been engineered to minimize utilization of various pantothenate biosynthetic enzymes and/or isoleucine-valine-biosynthetic enzymes and/or their respective substrates from being used to produce an alternative product identified as [R]-3-(2-hydroxy-3-methyl-butyrylamino)-propionic acid (HMBPA).

During the earlier stages of the present invention, experiments have been carried out which show that an unexpectedly high volumetric productivity can be achieved when using a semi-continuous fermentation process (SCP) for the production of pantothenate. A *Bacillus subtilis* strain with the genotype $P_{15}panBCD\ P_{15}\ panE$ (e.g. described in WO 01/21772) has been used for these experiments. Compared to prior art process designs for the pantothenate production like the standard fed-batch processes, the volumetric productivity could be improved by a factor of two. The semi-continuous fermentation process comprises fermentation in one tank with a variable feeding rate (f) and for example 10-25% medium withdrawals (by volume of the total fermenter working volume at the time of the medium withdrawal) at specific time intervals. Furthermore the withdrawn medium is collected in a separated buffer tank and then transferred to downstream processing.

Based on this semi-continuous process, we unexpectedly found that a semi-continuous cascade system according to the present invention unexpectedly allows to further improve $Y_{P/S}$ and $P_V$ in the pantothenate production. This is shown in two examples below. The first example aimed at improving the performance indexes by partially separating cell growth and pantothenate synthesis, thus an increased pantothenate yield was achieved at the expense of biomass formation. This also resulted in a higher productivity. Such improvements accounted for ~28% and ~33%, compared to SCP, in $Y_{P/S}$ and $P_V$, respectively. In the second example a dynamic approach for culture (medium) transfer and removal was applied. This approach together with the application of an optimum feeding range resulted in ~40% and ~60% increase in $Y_{P/S}$ and $P_V$, respectively, compared to SCP.

Furthermore the present invention allows to improve the conversion of glucose into pantothenate. This is a result of the extra biomass which is transferred to the next fermentation tank in order to extend its biocatalitic activity before being harvested. This operating mode can additionally be combined with glutamate co-feeding, which in the presence of ammonia ions, derived from the pH control, allows an optimum modulation of Carbon/Nitrogen fluxes in favour of pantothenate production. In general the benefit of this design can be applied to any bioprocesses targeting the production of other vitamins or fine chemicals.

Other multistage configurations for fermentation processes have been reported in the literature (Coodijer et al., Enzyme and Microbial Technology (1996); 18: 202-219; Kazuyuki et al., Chem. Eng. Comm. (1987); 52: 61-74; Kulozick et al., J. Biotechnol. (1992); 22: 107-116.). But most of these studies refer to Chemostat operations in STRs (stirred tank reactors) and/ or try to mimic plug-flow systems, in which the number of STRs may sometimes reduce the overall productivity due to the larger holding time of the series as compared to a single CSTR (Chemostat stirred tank reactor). In the semi-continuous cascade system according to the present invention, the STRs operate independently as production tanks, although they are connected for biomass transference. Another difference is that the culture transfer procedure is managed in intermittent fashion.

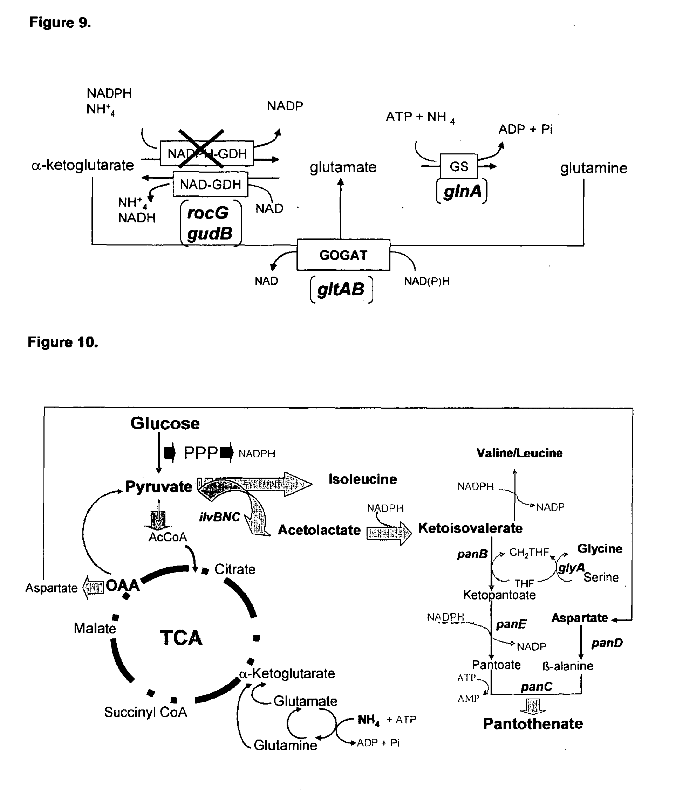

The starting point for the present invention was therefore a semi-continuous process as described above. Using different microorganisms, it was found that the more biosynthetic gene elements were engineered and /or manipulated to improve the carbon flux into the pantothenate pathway (Figure 12), the slower was the growth of these strains, reducing the rate of pantothenate production. A simple explanation of these results is that cell growth and pantothenate production were competing for the same central metabolism intermediates (Figure 10 and Figure 12).

To solve this problem, a compensation of the slower growth and production rates by oversupply of a complex nitrogen source, e.g. Cargill's soy flour (WO 02/061108 A2) was found not to be appropriate for food applications due to the high amount of impurities in the soy flour. If CalPan for food applications is the target, a cleaner fermentation broth is required. Therefore the present invention provides an alternative bio-processing method which optimizes the pantothenate production (i.e. product yield and productivity) using a cleaner fermentation broth suitable for CalPan food applications. This fermentation is referred to as semi-continuous cascade system (SCCS) for the CalPan production.

The present invention refers to a method for producing pantothenate by a semi-continuous cascade fermentation process, preferably using a pantothenate producing microorganism, further preferred using a pantothenate overproducing microorganism, wherein

a) the process is performed in at least 2 fermentation tanks ($T_1$, $T_2$, to $T_n$), which are interconnected in a row and additionally it is preferred that the tanks comprise the same initial working volume;

b) medium transfers ($V_T$) are performed for all tanks except for the last tank and the medium transfers are performed from one tank to another in the direction from the first to the last tank;

c) medium withdrawals ($V_W$) are performed for all tanks, and the medium withdrawals are performed into a separated buffer tank, preferably medium transfers and/ or medium withdrawals are performed at constant time intervals;

e) the constant and/or variable feeding rate ($f_n$) can be specific for each tank ($T_1$, $T_2$, ...$T_n$), but preferably $f_n$ is the same for all tanks.

f) F is the total volume of feeding solution for each tank $T_n$, provided during the whole fermentation process and F defines the amounts of medium to be withdrawn and/ or transferred from the tanks $T_n$ as:

$$F_n\ (l) = (\text{total volume of withdrawn medium from tank } T_n\ (l)) + (\text{total volume of transferred medium out of tank } T_n\ (l)) - (\text{total volume of transferred medium into tank } T_n\ (l)).$$

In a preferred embodiment of the method, all the medium withdrawals and all the medium transfers comprise to remove the same amount of medium, wherein the amount of medium to be removed each time is 10-20 %, further preferred 15-20 %, of the initial starting volume of the fermentation tanks.

It is further preferred that the operation time of the fermentation process in the tanks $T_n$ is more than 60 hours.

In another preferred embodiment of the method, medium withdrawals and/ or medium transfers are performed more than 2 times for each tank $T_n$ during the fermentation process.

It is especially preferred that the time interval is less than 10 hours, further preferred less than 8 hours, most preferred less than 6 hours.

In another embodiment of the invention, the first medium transfer is not performed in the first 6 hours of the fermentation.

In an especially preferred embodiment of the method, the feeding solution comprises a concentration of $PO_4^{3-}$ ions to keep the $PO_4^{3-}$ ion concentration in the fermentation medium of the tanks $T_n$ at a level of 500 mg/l or higher.

In a further preferred embodiment of the method, the feeding solutions comprises the following ingredients at a minimum concentration of: $KH_2PO_4$, 6 g/l further preferred 10 g/l; $K_2HPO_4 \cdot 12H_2O$, 6 g/l, further preferred 10 g/l; $Na_2HPO_4 \cdot 12H_2O$, 12 g/l, further preferred 16 g/l.

It is further preferred that the dissolved oxygen ($pO_2$) level is kept at $\geq$ 15%.

It is also preferred that the feeding rate ranges from 12 g/l/h up to 41 g/l/h.

In another preferred embodiment of the method according to the present invention, the ratio of the initial starting volume in one of the fermentation tanks $T_n$ / total amount of withdrawn medium from this tank $T_n$ is in the range of 3:1 - 1:2, further preferred 1:1 - 1:2.

It is further preferred that the feeding solutions comprises at least 32 g/l glutamate further preferred 45 g/l glutamate.

In a especially preferred method, at least 2 runs, further preferred at least 4 runs, even further preferred at least 6 runs and most preferred at least 10 runs, are performed during the whole fermentation process, wherein a run comprises one medium withdrawal and/or one medium transfer out of each tank $T_n$.

The specific parameters for the semi-continuous cascade system and the fermentation process according to the present invention are described in more detail below.

The semi-continuous cascade system can be carried in any containments such as stirred tank reactors or any known containments suitable for culturing. The containments can be chosen depending on the scale of the process. Additionally the containments should be suitable for providing a continuous feeding rate (f) and allowing a discontinuous medium withdrawal and/ or medium transfer and the necessary stirring or shaking of the culture. The size of the containments depends on the scale of the pantothenate production. It is preferred to use containments with a volume for the medium of at least 50 m$^3$, more preferred at least 100 m$^3$ and even more preferred at least 150 m$^3$ and most preferred between 150 m$^3$ and 220 m$^3$. It is further preferred to use stirred tank reactors. Preferably at least two tanks, further preferred at least 3 tanks, even further preferred at least 4 tanks and most preferred at least 5 tanks are used for the fermentation process and are interconnected in a row. The expression "interconnected" means that a specific amount (by volume) of fermentation medium can be transferred at a specific point of time from one tank to the next tank in row. The interconnected tanks are named as $T_n$ ($T_1$, $T_2$, ...), wherein the total number of tanks interconnected in a row defines the highest value of n in $T_n$. An (additional) separated tank is used to collect the withdrawn media, taken from the tanks.

At the beginning, all (in a row) interconnected tanks $T_n$ are filled with an initial starting volume of an inoculum preparation and medium (further details are provided in the examples). Additionally the initial starting volume of fermentation medium is preferably the same in all tanks $T_n$. Then the fermentation is carried out with a constant or variable feeding rate provided for each of the tanks $T_n$. The feeding rate ($f_n$) can be specific for each tank $T_n$, but preferably the feeding rate ($f_n$) is the same for all tanks $T_n$ as it is described in more detail below. The feeding rate provides the total volume $F_n$ (in liter, l) of feeding solution over the whole operation time of the fermentation process for each tank $T_n$. The volume of fermentation medium in each tank $T_n$ during the process and at the end of the fermentation process is the initial starting volume +/- 10 %, which is discussed in more detail below.

The term "medium transfer" as used in the context of the present invention defines to remove at once (discontinuous) a specific amount of medium ($V_T$) from one tank $T_n$ and add it to another tank $T_{n+1}$ which is next in the interconnected row of fermentation tanks $T_n$. The term "medium transfer" also defines to receive at once (discontinuous) a specific amount of medium from one tank $T_n$. The medium (culture) transfer is performed from the first tank $T_1$ to the second tank $T_2$ and from the second tank $T_2$ to the third tank $T_3$ and so on, depending on the numbers of tanks (interconnected in a row) used in the fermentation process. No medium transfers are performed for the last tank. The medium transfers comprise to always transfer the same amount (by volume) of medium ($V_T$) from one tank to the next tank in a row. Preferably an amount (by volume) of 5-30 %, further preferred of 10-20 %, even further preferred 15-20 % of the initial starting volume of one of the fermentation tanks is transferred each time. $V_T$ is the same for all tanks $T_n$.

The term "medium withdrawal" or "withdrawals of medium" as used in the present invention defines to remove at once (discontinuous) a specific amount of medium $V_W$ (by volume) from one tank $T_n$ and add it to the separated (additional) tank. The withdrawn medium of all tanks $T_n$ is collected in the (additional) separated buffer tank. The medium withdrawals $V_W$ (in liter (l)) can comprise about the same amount (by volume) of medium as the medium transfers $V_T$, which is then about 10-20 %, preferably 15-20 %, of the initial starting volume of one of the fermentation tanks $T_n$. Furthermore the medium withdrawals from the last tank comprise to withdraw a higher amount of medium from this tank in order to reach the initial starting volume at the end of the fermentation process, as the last tank receives medium transfers but no medium is transferred out of the last tank.

Medium transfers and/ or medium withdrawals are performed at constant time intervals. A run starts at tank $T_1$ and continuous with the next tank in row. This means (considering that for example 3 tanks are used in a row) that medium is transferred from the first tank $T_1$ to the second tank $T_2$ at a chosen point of time. After a chosen time interval, medium is transferred from the second tank $T_2$ to the third tank $T_3$ and at the same time, medium is withdrawn from $T_2$ and added to the separated tank. After the next time interval, medium is withdrawn from tank $T_3$ (last tank in the row) and added into the separated tank. If medium was withdrawn and/ or transferred from every tank, one run is completed.

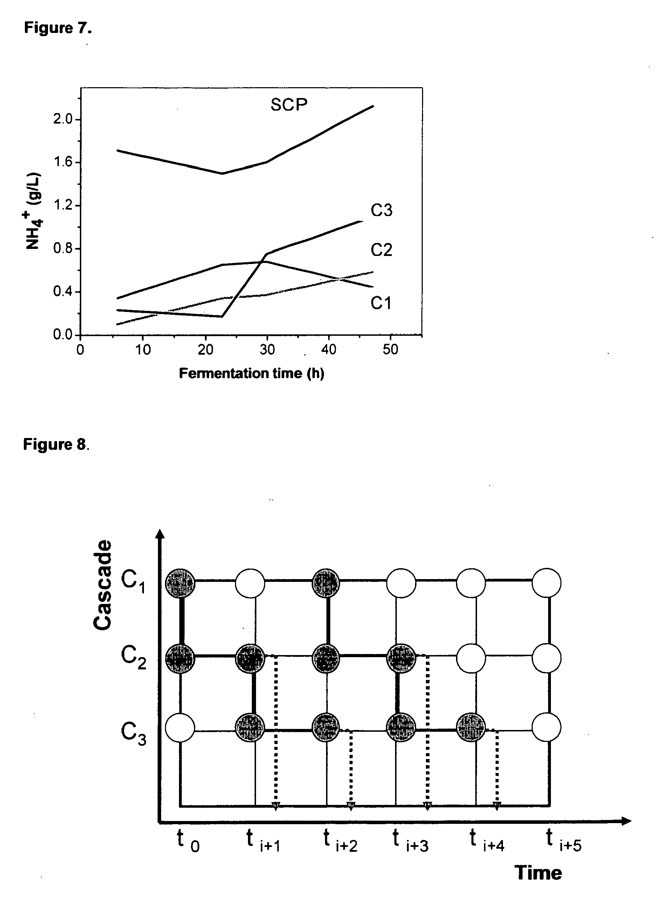

Then the next run starts after the following time interval or can preferably start simultaneously at an earlier time interval (simultaneous run). The simultaneous run comprises that at each point of time medium is withdrawn and/ or transferred from more than one tank (compare Figure 8), wherein only one run can start at one point of time.

It is preferred that at least 2, further preferred at least 4, even further preferred at least 6 and most preferred at least 10 runs are performed during the whole fermentation process.

The first run (which starts with a medium transfer from the first tank $T_1$ to the second tank $T_2$) starts after a first period of fermentation. The first medium transfer (start of the first run) is preferably not performed in the first 3 hours, further preferred not in the first 6 hours, and most preferred not in the first 8 hours of fermentation. The time intervals for the following medium withdrawals and/ or transfers are preferably smaller than 10 hours, further preferred smaller than 6 hours, even further preferred smaller than 4 hours.

The fermentation process for the production of pantothenate according to the present invention uses microorganisms which are pantothenate producers, preferably pantothenate overproducers. Preferred microorganisms are: *Bacillus, Corynebacterium, Lactobacillus, Lactococci, Streptomyces.* Preferably the microorganism is of the genus *Bacillus.* Preferable species of the genus *Bacillus* are: *Bacillus subtilis, Bacillus lentimorbus, Bacillus lentus, Bacillus firmus, Bacillus pantothenicus, Bacillus amyloliquefaciens, Bacillus cereus, Bacillus circulans, Bacillus coagulans, Bacillus licheniformis, Bacillus megaterium, Bacillus pumilus, Bacillus thuringiensis, Bacillus halodurans, Bacillus brevis* and *Bacillus stearothermophilus.*

The microorganisms which is especially preferred for the synthesis of pantothenate is *Bacillus subtilis.* It is further preferred that the *Bacillus subtilis* strain overexpresses at least two pantothenate biosynthetic enzymes. It is even further preferred that at least two pantothenate biosynthetic enzymes selected from the group consisting of *panB, panC, panD* are overexpressed. It is especially preferred that the *panB, panC* and *panD* biosynthetic enzymes are overexpressed. It is further preferred that MTF is engineered (this is described in WO 2004/005525). Most preferably a *Bacillus subtilis* strain is used, having the genotype $P_{15}$ *panBCD* $P_{15}$ *panE.*

The microorganisms can be cultured in liquid medium. The term "culturing" includes maintaining and/or growing a living microorganism of the present invention. The culturing is performed under conditions to improve the productivity and product concentration while maintaining the strain capability of glucose conversion into pantothenate. The parameters like temperature, pressure and pH are analog to fermentation processes known in the art (see US 2005/0124030), wherein it is preferred to control the temperature at 30-50°C, further preferred 37-40°C, more preferred at 38.5-39.5°C. Preferably the pH is kept at 6-8, further preferred $6.8 \pm 0.5$ and even more preferred at $6.8 \pm 0.1$. In addition it is preferred to control the pH-value by addition of $NH_4OH$ 27%.

In addition, the semi-continuous cascade system can be used for fermentation processes with operation times of more than 48 hours, preferably more than 100 hours, even more preferred for more than 120 hours and most preferred for more than 140 hours. The operation time in this context is defined as the fermentation time in the main fermentation tanks $T_n$ (reactors, which are interconnected in a row).

The microorganisms of the present invention are cultured under controlled aeration. The term "controlled aeration" includes sufficient aeration (e.g., oxygen) in all the tanks $T_n$ to result in the production of the desired product. It is preferred that air is sparked through the fermentation containment, preferably the air is sparked at $\geq 1$ vvm (volume of air per volume of medium per minute. The dissolved oxygen partial pressure ($pO_2$) can be measured during the fermentation process, e.g. with polarographic oxygen probes. The agitation speed and aeration can be used to control the $pO_2$, wherein a stepwise increase of the agitation speed might be necessary to control the $pO_2$ at preferable values $\geq 10\%$, more preferably $\geq 15\%$.

Additionally, it is preferred that the level of pantothenate production is at a level of more than 25 g/l and further preferred more than 34 g/l, before the first withdrawal of medium is performed.

Appropriate culture medium (or production medium) can be chosen by a person skilled in the art. An exemplary culture medium is given in the examples. The basal feeding solution used for the semi-continuous cascade system comprises glucose preferably in an amount of 500-800 g/l, further preferred 700-800 g/l. In addition the feeding solution preferably contains $MgSO_4 \cdot 7\,H_2O$, at least 2 (g/l); $MnSO_4 \cdot H_2O$, at least 0.015 (g/l) and $ZnSO_4 \cdot 7\,H_2O$ at least 0.004 (g/l).

Additionally preferably the basal feeding solution is supplemented with additional $PO_4^{3-}$ ions and glutamate. In one embodiment of the invention the concentration of $PO_4^{3-}$ ions in the feeding solutions is adjusted to keep the $PO_4^{3-}$ ion concentration in the fermentation medium at a level of 500 mg/l or higher. The feeding solution furthermore comprises 32 g/l glutamate. Additionally the feeding solution comprises preferably $(NH_4)_2SO_4$; 2.5 (g/l), and a mixture of trace elements similar to that used in the main culture.

In another embodiment of the invention, the feeding solution comprises the following ingredients at a minimum concentration of: $KH_2PO_4$, 6 g/l; $K_2HPO_4 \cdot 12H_2O$, 6 g/l; $Na_2HPO_4 \cdot 12H_2O$, 12 g/l. The feeding solution furthermore comprises 32 g/l glutamate.

Additionally the feeding solution comprises preferably $(NH_4)_2SO_4$, 2.5 (g/l), and a mixture of trace elements similar to that used in the main culture.

The feeding in each fermentation tank is preferably performed at a continuous rate. The feeding rates ($f_n$) can be specific for each tank, depending on the production target. The feeding rate ($f_n$), the media withdrawals $V_W$ and the media transfers $V_T$ are calculated so that the volume (in the tanks $T_n$) during the process and at the end of the fermentation process is the initial starting volume +/- 10 % in these tanks, preferably the volume in the tank $T_n$ is the initial starting volume +/- 3 %, whereas it is not necessary that the initial starting volume is the same for all the tanks $T_n$. Furthermore the total amount of feeding solution by volume (l) for each tank $T_n$, provided during the whole fermentation process, is defined as feeding volume ($F_n$).

The relation between F and the amount of medium by volume which is withdrawn from a tank and the amount of medium which is transferred out of a tank and the amount of medium transferred into the tank is given in Equation (1) below. Equation (1) results from the assumption that the volume (in the tanks $T_n$) at the end of the fermentation process is the same as the initial starting volume in these tanks.

The total volume withdrawn from a tank $T_n$ is calculated as the product of: (number of runs) x $V_W$. Furthermore the total volume of medium transferred out of a tank is calculated as the product of: (number of runs) x $V_T$ (transferred out of the tank). And the total volume of medium which a tank $T_n$ receives during the whole fermentation process is defined as the product of: (number of runs) x $V_T$ (which the tank receives).

$$F \text{ (total volume of feeding solution for each tank } T_n \text{ in liter (l))} = \text{(total volume of withdrawn medium from tank } T_n \text{)} + \text{(total volume of transferred medium out of tank } T_n \text{ (in liter (l)))} - \text{(total volume of transferred medium into tank } T_n \text{ (in liter (l)))}. \quad \text{Equation (1)}$$

The feeding rate is defined by the need of controlling bacterial growth ($\mu$) within the range of 0.02 and 0.07 h$^{-1}$.

Equation (1) and the feeding rate can be used to calculate the medium withdrawals $V_W$ and the medium transfers $V_T$.

An exemplary calculation for a process according to the present invention is given in the following: At the beginning of the fermentation process using the semi-continuous cascade system, for example the number of runs can be chosen. Furthermore the volume $V_T$ is chosen e.g. as 20% of the initial starting volume of tank $T_1$. Then Equation (1) allows to calculate F, because no withdrawals are performed for Tank $T_1$ and the product: (number of runs) x $V_T$ = total volume of transferred medium out of tank $T_1$ (l). For the last tank, Equation (1) defines the total volume of withdrawn medium as: F + total volume of transferred medium into the tank. $V_W$ for the last tank can then be calculated considering the number of runs. Accordingly, considering the number of runs, $V_W$ to be withdrawn from the other tanks (first and last tank have already been calculated above) can be calculated from Equation (1) as: F = total volume of withdrawn medium from tank $T_n$ (as the same amount of medium is received and transferred). It is understood that the present invention is not restricted to this exemplary calculation.

Another additional feature of this system is the possibility of modulating $V_T$:$V_W$ in a way that the biocatalitic activity of the fermented broth is extended for a given period of time in the next reactor instead of being withdrawn for recovery. Furthermore, $V_T$ is constrained by the feeding rate in $T_n$ and the working volume in the reactor, and hence withdrawals are applied. All the medium which has been withdrawn from the fermentation containment is collected in another separated buffer tank and transferred to downstream operations.

Additionally the present invention includes a step of recovering the desired compound (e.g., pantothenate). The term "recovering" a desired compound includes extracting, harvesting, isolating or purifying the compound from the culture medium. In order to achieve high amounts of pantothenate, the culture medium of the main fermentation tank and the withdrawn medium is used. The overall yield is based on the average of the main and the buffer tank.

Recovering (harvesting) the compound can be performed according to any conventional isolation or purification methodology known in the art including, but not limited to, treatment with a conventional resin (e.g., anion or cation exchange resin, non-ionic adsorption resin, etc.), treatment with a conventional adsorbent (e.g., activated charcoal, silicic acid, silica gel, cellulose, alumina, etc.), alteration of pH, solvent extraction (e.g., with a conventional solvent such as an alcohol, ethyl acetate, hexane and the like), dialysis, filtration, concentration, crystallization, recrystallization, pH adjustment, lyophilization and the like.

For example, a compound can be recovered from culture medium by first removing the microorganisms from the culture. Medium are then passed through or over a cation exchange resin to remove cations and then through or over an anion exchange resin to remove inorganic anions and organic acids having stronger acidities than the compound of interest.

Preferably, a desired compound of the present invention is "extracted", "isolated" or "purified" such that the resulting preparation is substantially free of other medium components (e.g., free of medium components and/or fermentation byproducts). The language "substantially free of other medium components" includes preparations of the desired compound in which the compound is separated from medium components or fermentation byproducts of the culture from which it is produced. In one embodiment, the preparation has greater than about 80% (by dry weight) of the desired compound (e.g., less than about 20% of other medium components or fermentation byproducts), more preferably greater than about 90% of the desired compound (e.g., less than about 10% of other medium components or fermentation byproducts), still more preferably greater than about 95% of the desired compound (e.g., less than about 5% of other medium components or fermentation byproducts), and most preferably greater than about 98-99% desired compound (e.g., less than about 1-2% other medium components or fermentation byproducts). When the desired compound has been derivatized to a salt, the compound is preferably substantially free of chemical contaminants associated with the formation of the salt. When the desired compound has been derivatized to an alcohol, the compound is preferably further free of chemical contaminants associated with the formation of the alcohol.

In an alternative embodiment, the desired compound is not purified from the microorganism, for example, when the microorganism is biologically non-hazardous (e.g., safe). For example, the entire culture (or culture supernatant) can be used as a source of product (e.g., crude product). In one embodiment, the culture (or culture supernatant) is used without modification. In another embodiment, the culture (or culture supernatant) is concentrated. In yet another embodiment, the culture (or culture supernatant) is dried or lyophilized.

To "harvest" the fermentation product comprises to harvest the fermentation medium from all tanks $T_n$ and the separated tank. The overall yield of the fermentation process using the semi-continuous cascade system can be determined as the total amount of product obtained (harvested) from all tanks $T_n$ and from all the withdrawn medium collected in the separated tank.

In order to determine the improved product concentration of the fermentation process according to the present invention, the pantothenate titer can be measured in the reaction tanks $T_n$ and in the separated tank containing the withdrawn medium. To determine the pantothenate titer can be performed using known methods in the art (see Experiment 9) Preferably the overall product concentration is greater than 25 g/l, further preferred greater than 30 g/l, even further preferred greater than 35 g/l and even further preferred greater than 80, further preferred 100 and most preferred greater than 120 g/l.

## Description of the figures:

**Figure 1 (A and B).** Comparative time-course profile of pantothenate production indexes of a strain *Bacillus subtilis* having 4 or more than 4 pantothenate genes overexpressed using the SCP. This strain ($P_{15}$ *panBCD* $P_{15}$ *panE*) has been used for all fermentation experiments (WO 01/21772). **A** provides the evolution of the conversion efficiency index ($Y_{P/S}$) while **B** shows the product accumulation in the fermentation broth along the process time (titer).

**Figure 2.** Pantothenate specific productivity as a function of the glucose uptake rate.

**Figure 3.** Time course profile of $Y_{X/S}$ as compared to $Y_{P/S}$. Data integration was managed using polynomial fitting of raw data.

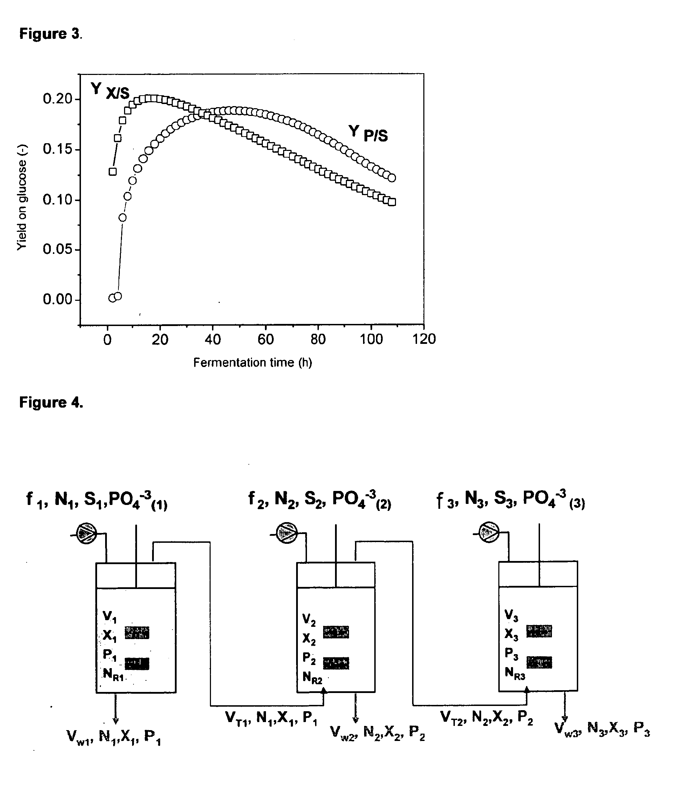

**Figure 4.** Schematic diagram of the cascade-tanks design. $f_1$, $f_2$,.. represent the feeding rates for the tanks $T_1$, $T_2$,..; $N_1$, $N_2$,.. represent the nitrogen source supply for the $T_1$, $T_2$,..; $N_{R1}$, $N_{R2}$, represent the nitrogen levels in the tanks $T_1$, $T_2$,..; $S_1$, $S_2$,.. represent the glucose supply through the feeding solution for the tanks $T_1$, $T_2$,..; $PO_4^{3-}{}_{(1)}$, $PO_4^{3-}{}_{(2)}$,.. represent the $PO_4^{3-}$ supply through the feeding solution; $V_1$, $V_2$,.. represent the volumes in the tanks $T_1$, $T_2$,..; $V_{T1}$, $V_{T2}$,.. represent the transfer volumes from the tanks $T_1$, $T_2$,..; $X_1$, $X_2$,.. represent the biomass in the tanks $T_1$, $T_2$,..; $P_1$, $P_2$,.. represent the product concentration in the tanks $T_1$, $T_2$,.. and in the transferred medium.

**Figure 5.** Feeding rate for each STR in the cascade system C1 (refers to tank $T_1$), C2 (refers to tank $T_2$), C3 (refers to tank $T_3$).

**Figure 6.** Time course profiles of pantothenate production of Example 12. Figure 6 shows product concentration, product output and comparative biomass evolution (DCM refers to the <u>D</u>ry <u>C</u>ell <u>M</u>ass) in each tank comprising the cascade reaction system. The process design promotes the growth in $STR_1$ (cascade 1) and production in $STR_2$ and $STR_3$ (cascade 2 and cascade 3, respectively).

**Figure 7.** Follow-up of $NH_4^+$ accumulation in the fermentation medium. SCP is the control run carried out with 3 withdrawals using the semi-continuous fermentation process, while C1 refers to tank $T_1$, C2 refers to tank $T_2$, C3 refers to tank $T_3$.

**Figure 8.** Stepwise approach of medium transfer and withdrawals (C1 refers to tank $T_1$, C2 refers to tank $T_2$, C3

refers to tank $T_3$). Dynamic programming of fermentation broth management over the fermentation time course of 2 simultaneous runs in cascade. Dashed lines indicate withdrawals, thick lines indicate medium transfers.

**Figure 9.** Central Nitrogen Metabolism (CNM): Interconversion of $\alpha$-ketoglutarate, ammonia, glutamate and glutamine in the CNM by enteric-bacteria and yeasts (Schure ter et al, Microbiol. Rev. (2000); 24: 67-83.). Ammonia can be converted into glutamate via two pathways. It may be coupled directly to $\alpha$-ketoglutarate to form glutamate by NADPH-GDH (nicotinamide adenine dinucleotide phosphate - glutamate dehydrogenase), a reaction which costs NADPH (nicotinamide adenine dinucleotide phosphate). It may also be incorporated into glutamine via GS (glutamine synthetase) at the expense of an ATP (adenosine triphosphate). Glutamine and a-ketoglutarate are converted to glutamate via GOGAT (glutamate synthase) at the expense of NADH. Glutamate can be degraded to $\alpha$-ketoglutarate and ammonia via NAD-GDH using NAD. GDH in Bacillus subtilis (GlutDH, glutamate dehydrogenase) is exclusively a catabolic enzyme as indicated below (Belitsky et al., J. Bacteriol. (1998); 180: 6298-6305; Belitsky et al., J. Bacteriol. (2004); 186: 3399-3407.). The CNM encoding genes identified in Bacillus subtilis are depicted in brackets below the respective enzyme reactions (Kunst et al., Nature (1997); 390: 249-256), rocG and gudB are genes encoding glutamate dehydrogenase; Pi = inorganic phosphate.

**Figure 10.** Key dependences of pantothenate synthesis on the central metabolism pathway. Further definitions: AcCoA (acetyl coenzyme A), TCA (tricarboxylic acid cycle), THF (tetrahydrofolate), ATP (adenosine triphosphate), AMP (adenosine monophosphate), OAA (oxalic acid).

**Figure 11.** Computing solution of the dynamic approach for managing culture transference and withdrawals. Equation (1) was implemented to predict $V_T$ and $V_W$.

**Figure 12.** Metabolic Pathway of Pantothenate synthesis with involved enzymes and genes coding therefore.

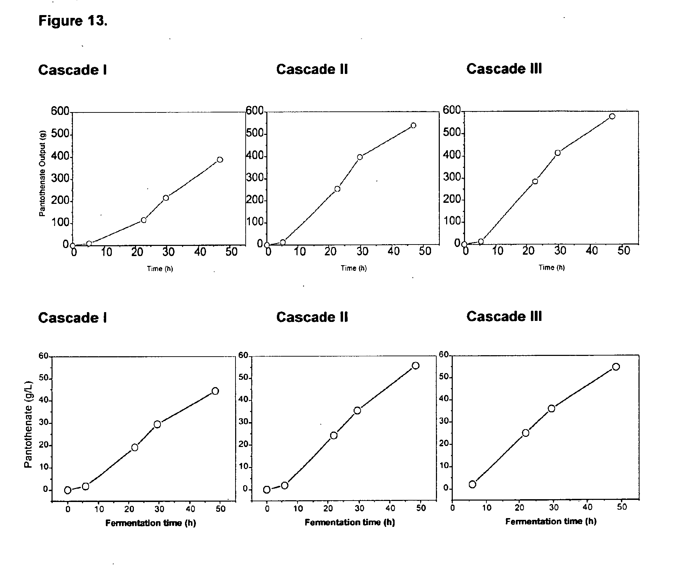

**Figure 13.** Time course profile of pantothenate production of Example 13. Figure 13 shows the product concentration (titer) and product output.

**Experiment 1: Analysis of prior art processes**

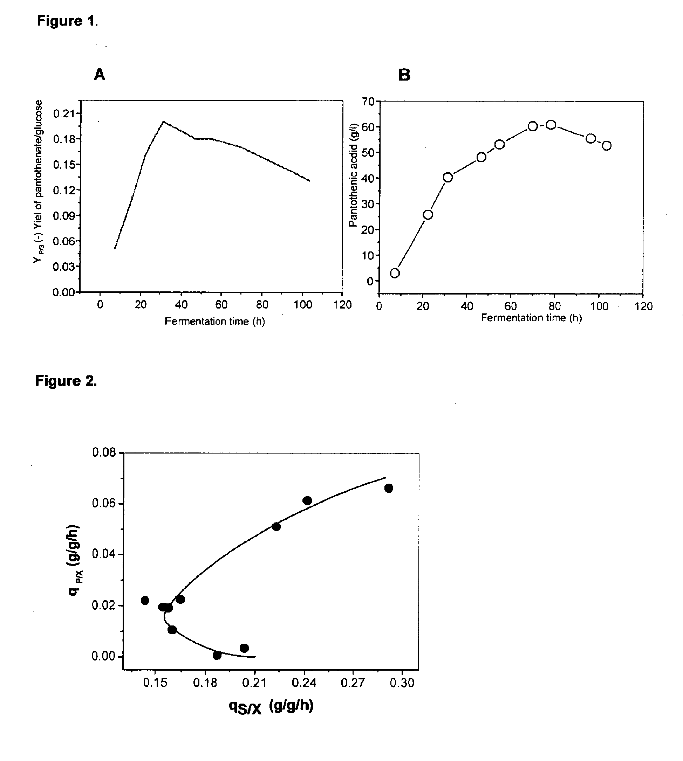

It has been noticed that as the number of over-expressed pantothenate biosynthetic genes increases, the pantothenate biosynthetic capability of *Bacillus subtilis* increases but the growth performance is diminished (Fig. 3). High rates of pantothenate production depend therefore on rich medium. This particular requirement can not be fulfilled by semi-continuous process where yeast extract is depleted within the first 5h and most of the fermentation is carried out using minimum medium. Furthermore, media enrichment by additional complex nitrogen sources such as soy flour (as applied in WO02/061108) may provide technical solutions at fermentation level but would bring about additional complications in the recovery and purification of pantothenate, especially for food application purposes. As shown in Fig. 1A, the pantothenate yield of a strain having more than 4 pantothenate genes over-expressed starts quickly peaking at around 30h and then slows down dramatically towards the end of the fermentation when grown under semi-continuous mode operation (SCP). Product accumulation occurs not only at expenses of high Carbon-inflow but also shows a decay pattern as time progresses (Fig. 1B). To achieve the full potential of this strain, as seen during the first 30 h of cultivation, a different cultivation approach may have to be introduced. To this end a set of evaluations were performed aiming at understanding the above depicted metabolic behavior.

First of all the specific pantothenate synthesis *versus* glucose supply was reviewed. After the threshold $q_{s/x}$-level (specific substrate uptake rate (g substrate/ g biomass/ h)) was reached (0.14 $gg^{-1}$ $h^{-1}$) in the second phase of the fermentation phase, glucose supply was increased by ~40% per biomass unit ($q_{S/X}$ up to ~0.20 $gg^{-1}h^{-1}$) as can be seen in Figure 2. As a result, the specific pantothenate synthesis productivity ($q_{P/X}$) moved downwards and not upwards as expected (Figure 2). These results show that under the given conditions, shifting back to the optimum $q_{S/X}$ does not recover the initial performance.

A closer observation of the biomass generation as compared to pantothenate synthesis revealed the performance of the strain (Figure 3). Especially, the biomass yield ($Y_{X/S}$) break-down occurs much earlier than pantothenate yield and keeps diminishing at a constant rate all the way up to the end of the fermentation. Some level of break-down in $Y_{X/S}$ is expected because of a switch from growth to pantothenate production, but a negative slope is not desired.

This $Y_{X/S}$ pattern brings into question the effect of N-source and medium formulation on growth and pantothenate production. N-supply in SCP follows pH control using $NH_4OH$ in lab scale. $NH_4^+$ demand is around double than actually required for pantothenic acid neutralization, thus N-limitation appears not to be the case. It has been observed however that free $NH_4^+$ accumulates in the medium at high concentrations (> 5 g/l) even when the glucose supply is increased. In medium containing glucose and ammonium the assimilation of $NH_4^+$ seems to be restricted to glutamine synthetase mediated by GlnA (glutamine synthetase , specified as GS in Figure 9) because *Bacillus subtilis* has no assimilatory glutamate dehydrogenase (GlutDH) activity (Fisher et al., Mol. Microbiol. (1999); 32: 223-232.). GlutDH encoded by *rocG* and *gudB* are exclusively catabolic enzymes (Belitsky and Sonenshein, 1999).

When nitrogen is in excess, glutamine and other inhibitors of GS accumulate and bind to the enzyme, inactivating it as an enzyme and causing it to form a complex with TnrA (TnrA = global nitrogen-regulatory protein), a global nitrogen-regulatory protein (Belitsky et al., J. Bacteriol. (2004); 186: 3399-3407.). The feedback inhibition of GS may also prevent the cells direct access to glutamate. Glutamate is synthesized by the only reaction of glutamate synthase (*gltAB*) requiring glutamine, $\alpha$-ketoglutarate, and NADPH (Figure 9).

**Experiment 2: The Design-Concept**

It is the aim of this process design to improve $Y_{X/S}$, $Y_{P/S}$, and volumetric productivity ($P_V$) by managing both, medium formulation and mode of operation. This was done by:

- Medium formulation

    a) providing an additional defined nitrogen source
    b) increasing the level of trace elements

- Mode Operation

    a) Modifying the SCP to a cascade mode utilizing interconnected stirred tank reactors (Figure 4).

**Experiment 3: Medium formulation**

Glutamate was selected as an additional N-source because it plays an essential role in the central nitrogen metabolism and together with glutamine serves as nitrogen donor for all other nitrogen containing compounds in the cell. The partial replacement of $NH_4^+$ with glutamate results in energetic advantages; it saves the NADPH (nicotinamide adenine dinucleotide phosphate) needed for its synthesis and will provide NADH (nicotinamide adenine dinucleotide) when assimilated into the TCA cycle via $\alpha$-ketoglutarate (Figure 9). Furthermore, glutamate ought to relieve metabolic stresses created by both, the saturation of ammonium uptake and the down-regulation of GS.

It has been shown that glutamate uptake follows a simple transport system and its uptake rate is the highest one among other amino-acids tested using *Bacillus* membrane vesicles (De Vrij et al., J. Bacteriol. 1989). By using glutamate, a cheap and readily available N-source, a better balance between N-supply and N-uptake was achieved as can be judged by the lower accumulation of ammonia in the media (Fig. 7). The easier incorporation of glutamate into the TCA cycle and the synthesis of $\beta$-alanine (the second building block of pantothenate, Figure 10) resulted in an improved biomass yield ($Y_{X/S}$) as well as product yield ($Y_{P/S}$).

Other medium re-adjustments tested included an increase of trace elements as a function of the feeding volume. The $PO_4^{3-}$ threshold level in the fermentation medium was increased from ~0.25 to ~1.00 g/l. To manage this, the J solution (see Example 6) was provided right from the beginning of the feeding activation. To enable an easy handling of the changes introduced, the glutamate stock solution and the J solution were mixed with the glucose feeding solution after sterilization.

**Experiment 4: Mode of Operation (Cascade)**

The mode of operation of the SCP-Cascade relies in the intermittent medium withdrawals as in SCP but interconnects three stirred tanks reactors (STR) in a row (Figure 4). Such interconnection allows extending the metabolic activity of the biomass for an additional $\Delta t$ before being harvested. The setting design of the reactors is indicated in Figure 4. Key features of this design are:

- In principle any of the variables depicted ($f_n$, $N_n$, $S_n$, $V_n$, $X_n$, $V_{Wn}$, $V_{Tn}$) can be used as state or control vectors depending upon the performance index that is being targeted.
  Consequently an array or alternatives are exposed, which makes this system highly flexible.
- Although connected, each STR can have unique properties and specific optimization criteria.
- The culture transference ($V_{Tn}$) reduces the non-productive collection time of the withdrawals ($V_{Wn}$) by transferring active biomass to the next STR and allowing additional reaction time ($\Delta t$) before is harvested for downstream operations.

For the purpose of evaluating this fermentation design, the following variable settings were applied:

- Fermentation was carried out in all three STRs simultaneously
- Initial working volume was the same in all three reactors: $V_1(t_0) = V_2(t_0) = V_3(t_0)$
- Initial cell density was the same in all the STRs: $X_1(t_0) = X_2(t_0) = X_3(t_0)$
- Each tank was inoculated independently, but within the same time-frame
- One single medium formulation was used for the feeding solution in all STRs. It contained mainly C/N/P-sources.
- A rapid draw-off case was applied for the optimal culture removal and transference from one STR to the next one in order to minimize disturbances and optimize reaction time.

Taking into account all the multiple dependences depicted in Figure 4 and having $Y_{P/S}$, $Y_{X/S}$, and $P_V$ as optimization targets, it seemed reasonable to control the performance indexes by dynamic variation of feeding rate, working volume, size and timing of culture transference as well as withdrawals. In the following, two examples are provided which show the outcome of these approaches.

**Experiment 5: Strain**

Pantothenate overproducing *Bacillus subtilis* strain in which at least 4 pantothenate biosynthetic genes are overexpressed using a SPO1 phage promoter (as described, e.g. in WO 01/21772).

**Experiment 6: Culture Medium**

The composition of **VYS** medium is as follows (g/l): **V**eal infusion broth, 30; **Y**east extract, 5; **S**orbitol, 10; $K_2HPO_4$ 2.5. VYS medium was used in the first stage of the inoculum.

In the second stage inoculum and in the main culture the following medium formulation was applied (g/l): glucose, 40; Yeast extract, 16; Na-glutamate, 1; $KH_2PO_4$, 6.28; $K_2HPO_4$, 6.28; $Na_2HPO_4 \cdot 12H_2O$, 11; $NH_4Cl$, 0.31; $(NH_4)_2SO_4$, 1.88, Antifoam Basildon, 0.2; $MnSO_4 \cdot H_2O$, 0.017; $CoCl_2 \cdot 6 H_2O$, 0.005; $(NH_4)_6Mo_7O_{24} \cdot 4H_2O$, 0.0018; $AlCl_3$ 6 $H_2O$ 0.0012; $CuCl_2 \cdot 2H_2O$, 0.0009; $MgSO_4 \cdot 7H_2O$, 1.2; $ZnSO_4 \cdot 7 H_2O$, 0.005; $CaCl_2 \cdot 2 H_2O$, 0.0625; $FeSO_4 \cdot 7H_2O$, 0.05. Glucose and the trace elements were sterilized separately.

The basal feeding solution contains (g/l): Glucose, ~750-800; $MgSO_4 \cdot 7 H_2O$, 2; $MnSO_4 \cdot H_2O$, 0.015; $ZnSO_4 \cdot 7 H_2O$, 0.004.

For the two sets of SC-cascade fermentations reported in Examples 12 and 13, the basal feeding solution was supplemented with two additional solutions; namely the J solution in a ratio of 90 ml/l feeding, and the glutamate stock solution makes up to 8% of the feeding solution. The concentration of the glutamate in the stock solution is 400g/L while the composition of the J solution is as follows: $KH_2PO_4$, 90; $K_2HPO_4$, 90; $Na_2HPO_4 \cdot 12H_2O$, 164; $NH_4Cl$, 5; $(NH_4)_2SO_4$, 28, and a mixture of trace elements similar to that used in the main culture.

**Experiment 7: Inoculation Procedure**

Inoculum preparation was carried out in two stages. In the first stage 1 ml stock culture, previously prepared and preserved at -25°C, was inoculated into 25 ml VYS broth supplemented with tetracycline (15 ug/ml) in a 100 ml Erlenmeyer flask, which was then incubated for 7 h at 39°C on a rotary shaker at 200 rpm. In the second stage, 3 ml of this culture (1 % v/v) was transferred to 300 ml of the production medium containing 15$\mu$g/ml chloramphenicol. This second pre-culture was carried out in a 2 L Erlenmeyer flask and was incubated again at 39°C for 16 $\pm$ 1h, time at which an OD (optical density) > 14 was achieved. For the final stage, the content of such flask was transferred aseptically to the stirrer tank reactor (fermenter) to give approximately 5% w/w inoculum concentration.

**Experiment 8: Reactor and Operating Conditions**

Fermentations were carried out in a 15 l stirred tank reactor Fa. BBraun (Germany). The initial working volume was set-up at 6 l while the maximum volume depends upon the experiment in question. The temperature was controlled at 39°C, and the pH was kept at 6.8 $\pm$ 0.1 by controlled addition of $NH_4OH$ 27%. Air was sparged at $\geq$ 1 vvm, as required. The dissolved oxygen partial pressure ($pO_2$) was measured with polarographic oxygen probes. A back pressure of 500 mBar was applied. The initial agitation speed was set at 400 rpm with stepwise increase as required by the $pO_2$ control.

The $pO_2$ was controlled at values $\geq$ 15% by a combination of aeration and agitation using a PID controller action. This controller has one inner loop (a slave) involving the measurement of agitation speed and an outer loop (the master) involving the $pO_2$ measurement. The output of the $pO_2$ loop controller serves as the set point for the agitation loop. Once the agitation speed reached the upper limit (1000 $\pm$ 50 rpm), the control is switched to the aeration loop.

The aeration flow was adjusted in a stepwise form from 6 nL/min (throughput) to 12 nL/min as the lower limit of $pO_2$ was approached.

**Experiment 9: Determination Pantothenate and Pantothenate-Related Metabolites**

Chromatography of samples was performed on a Phenomenex LUNA C8 column, using an Agilent 1100 HPLC system equipped with a thermostatted autosampler and a diode array detector. The column dimensions are 150 x 4.6 mm, particle size 5 micron. The column temperature was kept constant at 20°C. The mobile phase is a mixture of 0.1 % acetic acid (A) and methanol (B). Gradient elution is applied, ranging from 1% B to 45% B in 15 minutes. The flow rate is 1 ml/min. Pantothenate was monitored using UV absorption at 220 nm, and is eluted at approximately 9.6 min. The calibration range of the method is from 1 to 100 mg/l pantothenate. Other pantothenate related metabolites are also separated and measured by this method.

**Experiment 10: Cell Dry Weight**

Cell dry weight concentration was determined in duplicate by gravimetric methods. The culture broth was diluted as required and then centrifuged in 2 ml Eppendorf tubes at 14000 rpm at 4°C for 10 min to settle cells. The supernatant was decanted and saved for other analytical determinations. The cell pellet was washed and then centrifuged again under the same conditions described for the sample centrifugation. After this second centrifugation the washing water was discarded and the cells pellet was dried under vacuum at 40°C and weighed.

**Experiment 11: Determination of Ammonium**

The determination of ammonium ions was done with an automatic analyser 'Vitros DT II" that works with special coated reaction slides. The slides are multilayered and contain immobilized/coated analytical elements on a plastic support. 10 $\mu$l of sample containing ammonium in the range of 1-500 $\mu$Mol was deposited on the slide and evenly distributed by the spreading layer. The ammonia in the sample then diffuses through a semi-permeable membrane into a layer containing bromphenol blue as the indicator dye. By measuring the amount of light reflected from the dyed layer after a fixed incubation period, the analyzer calculates the amount of ammonia present in the sample.

**Experiment 12: Use of SC-Cascade to Segregate Growth and Production**

Two sets of SC-cascade fermentations were performed in order to validate experimentally the concept-design as described in above.

The profiles of $Y_{X/S}$ and $Y_{P/S}$ in Figure 3 suggested that growth and production can be separated and that at a given time point one can be favored independently from the another one. In fact, $Y_{X/S}$ and $Y_{P/S}$ have their maxima at different time-points (Figure 3). These features combined with the cascade system may present different options to improve pantothenate fermentation. The design option chosen in this study relies on promoting growth in the first tank ($STR_1$) and production in the two others ($STR_2$, $STR_3$). $STR_1$ could then be used to transfer as much biomass as possible to $STR_2$ and $STR_3$, such that additional supply of active biomass would likely help to improve product yield and productivity in these tanks. In a sense, STR1 is targeted to become a "semi-continuous seed tank" that at the same time also operates as a production tank. Consequently, the constraints-setting in managing growth by N-supply was not expected to be trivial, especially when N- and C-sources are mixed in the feeding solution (see Experiment 3).

Although the mixed feeding solution established was kept as simple as possible, other challenges to the handling of $STR_2$ and $STR_3$ were still evident. For example, high feeding rates would not just increase C-supply but also N-supply, and hence biomass synthesis will be promoted. Consequently, a reduced feeding rate was evaluated as a means of avoiding too much biomass. Feeding reduction was managed by allowing working volume increase while keeping the feeding rate constant.

In general the same variable settings described in Experiment 4 were applied, however the following settings are specific to this experiment:

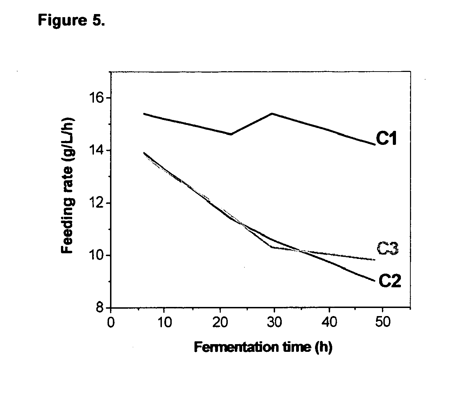

- Culture withdrawal allowed only at the end of the cascade: $\mathbf{V_{W\,(1)} = V_{W\,(2)} = 0}$
- Variable volume: $\mathbf{V_{R1} < V_{R2}, V_{R1} < V_{R3}, V_{R2} \equiv V_{R3}}$
- Variable feeding: $\mathbf{f_1 > f_2, f_1 > f_3, f_2 \equiv f_3}$ (Figure 5)
- Culture transference follows double purpose: a) from $STR_1$ to $STR_2$ $\mathbf{(V_{T(1)})}$ to maintain high feeding rates in order to promote growth, and b) from $STR_2$ to $STR_3$ $\mathbf{(V_{T(2)})}$ when $V_{max}$ becomes critical and to be able to achieve $\mathbf{V_{R2} \equiv V_{R3}}$. Table 1 shows a simplified transference schedule.

**Table 1.** Culture transference and withdrawals schedule

| Time | $V_{T (1)}$ (l) | $V_{T (2)}$ (l) | $V_{W (3)}$ (l) |
|---|---|---|---|
| 0 | | | |
| 5.3 | 0.8 | | |
| 5.5 | | 0.3 | |
| 22.7 | 0.8 | | |
| 23.0 | | 0.5 | |
| 29.8 | 0.8 | | |
| 30.2 | | 0.5 | |
| 47.0 | 0.8 | | |
| 47.2 | | 0.5 | 1.5 |

With the above described procedure, the application of the variables indicated in Figure 5 and Table 1, and keeping all other operating conditions as described in Examples 3-11, a clear improvement of the performance indexes set from economic targets could be achieved (Table 2). Furthermore, the $STR_1$ values as compared to those of $STR_2$ and $STR_3$ show unambiguously that growth could be segregated from production.

**Table 2.** Main performance indexes of a SC-Cascade fermentation process

| | Control SCP | $STR_1$ | $STR_2$ | $STR_3$ |
|---|---|---|---|---|
| $Y_{X/S}$ (-) | 0.17 | 0.26 | 0.17 | 0.19 |
| $Y_{P/S}$ (-) | 0.18 | 0.17 | 0.22 | 0.23 |
| $P_V$ (kg/m³/h) | 1.24 | 1.19 | 1.50 | 1.60 |

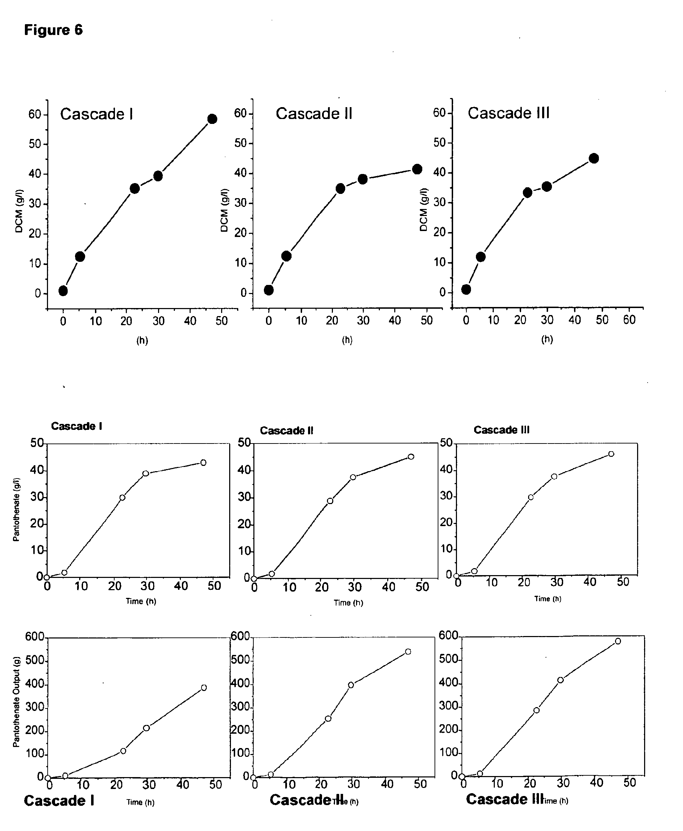

The supplementing of the feeding medium with additional glutamate together with the higher feeding rate per unit of working volume in $STR_1$ resulted in significantly higher biomass accumulation as compared to the other two tanks (Figure 6). The biomass yield increased from 0.17 in SCP to 0.26, resulting in 50% improvement.

During the fermentation, the demand of $NH_4OH$ per g of glucose feed remained unchanged when comparing $STR_1$ with SCP (~ 0.19 $\pm$ 0.0028 of 27% $NH_4OH$/g pure glucose). Similarly, the percentage of $NH_4OH$ used for pantothenate neutralization remained unchanged (~0.46 %) as judged by the yield of pantothenate on $NH_4^+$. Furthermore a significant reduction of $NH_4^+$ concentration in the fermentation medium in all three cascades (tanks) was observed as compared to the control run using semi-continuous recipe (Figure 7). These results indicate that the present invention provides a better utilization of the N-sources.

The above results are also in line with the categorization of ammonium as a "good N-source" able to support *Bacillus* growth relatively well, but not so for glutamate. Glutamate has been generally regarded as a poor N-source for *Bacillus* (compare above cited document: Fisher et al. (1993) Utilization of amino acids and other nitrogen-containing compounds. In Bacillus subtilis and other Gram-positive Bacteria: Biochemistry, Physiology, and Molecular Biology. Sonenshein, Hoch, and Losick, (eds). Washington, DC: American Society for Microbiology Press, pp.221-228.). Glutamine and not glutamine has been reported as the best N-source for *Bacillus* (Desth and Stülke, Microbiol. 2003).

This may be reasoned by looking at the glutamate catabolism pathway in *Bacillus* when growing cells in medium with glucose and glutamate as the single N-source. Glutamate before entering the glutamine pool has first to generate $NH_4^+$ on its own and may slow down the cellular pools resulting in slower growth. Conversely glutamine supply can offset the need for ammonium and can easily be converted to glutamate and initiate the glutamate/glutamine pool required for amino acid and nucleotides synthesis. However the kinetics of *Bacillus* growth in the presence of glutamate plus $NH_4^+$ has not been reported so far, the above results shows that such a combination can be beneficial.

Discrepancies on glutamate efficiency also rely on the cultivation mode and operating conditions used. Most of the N-screening studies reported in the literature have been carried out using agar plates and growth evaluation was based on qualitative observations (compare above cited document: Fisher et al. (1993)). The experiments with submerged cultures performed in course of the present invention show that despite the slower glucose uptake, glutamate can support *Bacillus* growth at rates similar to yeast extract and favour biomass formation at expenses of pantothenate production.

The overall production performance in the production tanks ($STR_2$ and $STR_3$) show clear improvements as compared to the "growth tank" ($STR_1$) as well as to the control run (SCP). The conversion efficiency of glucose into pantothenate ($Y_{P/S}$) and the volumetric productivities ($P_V$) were improved by around 28% and 33%, respectively (Table 2). Furthermore, the production capabilities of $STR_1$ are rather comparable to those depicted by the control run SCP (see $Y_{P/S}$ and $P_V$ in Table 2). Accordingly growth can be favoured without diminishing production.

**Example 13: Improving SC-Cascade Performance by Dynamic Approach**

The above described experiments indicate that the SC-cascade system is effective in improving pantothenate production by fermentation. Its versatility allowed different physiological conditions and metabolic activities demonstrating that growth and production can be induced independently as well as simultaneously. This is particularly important when dealing with partially growth associated compounds such as pantothenate. The above results also show that an optimum balance between growth and production provides the basis for higher conversion efficiency and enhanced production rates (e.g. $STR_3$, Table 2). Only a slight biomass yield improvement (~ 12%) in STR3 was enough to improve the substrate and time efficiency of pantothenate synthesis. Based on these observations, this Example focuses on further improvement in the performance of cascade system by managing C/N (Carbon/ Nitrogen) supply at levels that keeps central metabolism active but allows enough C for production. Furthermore, a dynamic programming approach was introduced to enable easier handling of the culture transfers and withdrawals.

To accomplish these two development objectives, the following design settings were applied:

- The feeding rate was set-up between the volumetric rates of C1 and C2/C3 in Figure 5.
  By finding an intermediate value, the reduction in feeding rates to avoid N-supply excess will be unnecessary. An initial upward trend will be aimed.
- No segregation of growth and production was pursued, thus all three STRs are used as "production tanks". Consequently all the feedings will be similar:

$$f_1 \approx f_2 \approx f_3$$

- A constant volume criterion was introduced to facilitate handling: $V_{R1} \equiv V_{R2} \equiv V_{R3}$
- To accomplish the previous condition (constant $V_R$) and having $f(C_n)$ fixed the withdrawals $(V_w)$ and transfers $(V_T)$ were calculated according to equation (1)
- To enable Equation (1), a dynamic programming of $V_w$ and transfers $V_T$ was developed and applied (Figure 8).
- All other variable settings and process design constraints were the same as described in Examples 3,4 and 12

The dynamic approach depicted in Figure 8 has the purpose of allowing enough time for biosynthetic activities once the culture transference takes place, yet at the same time enabling easy and coordinated culture handling. Accordingly, the SC-cascade system was treated as one single process that was divided into three steps C1, C2, and C3, while the time course of the process was divided into a number of stages $t_i$. At each time point ($t_i$) transfers and withdrawals of culture were taken, whereas between these time intervals metabolic activity of the newly transferred culture is allowed at rates set from the feeding pattern and the operating conditions.

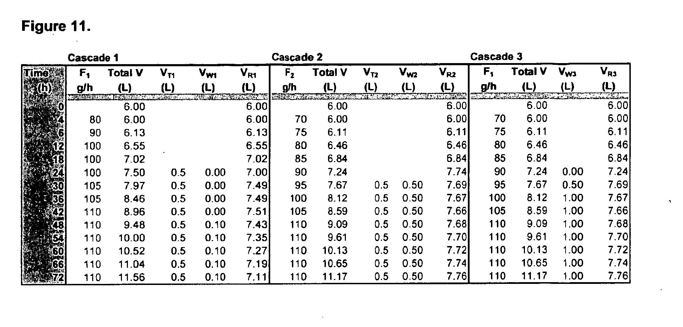

A computing solution of this approach is provided in Figure 11. Such profile was used as guide for scheduling manual culture transfers and withdrawals as shown in Table 3. Due to the lack of an automatic system longer time intervals had to be used. Moreover to improve accuracy of the volume transfers and withdrawals, an extra weight in the interconnection sections of the STRs was implemented.

**Table 3.** Dynamic programming of culture transfer and withdrawals for manual operation.

| Time (h) | $V_{T1}$ (L) | $V_{W2}$ (L) | $V_{T2}$ (L) | $V_{W3}$ (L) |
|---:|---:|---:|---:|---:|
| | | | | |
| 6 | 0.7 | | | |
| 9 | | 0.7 | 0.7 | |
| 12 | | | | 1.4 |

(continued)

| Time (h) | $V_{T1}$ (L) | $V_{W2}$ (L) | $V_{T2}$ (L) | $V_{W3}$ (L) |
|---|---|---|---|---|
| 20 | 0.7 | | | |
| 23 | | 0.7 | 0.7 | |
| 26 | | | | 1.4 |
| 28 | 0.7 | | | |
| 31 | | 0.7 | 0.7 | |
| 34 | | | | 1.4 |

With the above described procedure and variable settings, the application of the dynamic approach indicated in Table 3 & Figure 11, and keeping all other operating conditions as described in Examples 3-11, a further improvement of pantothenate production was accomplished (Table 4). All three tanks outperformed not just the control run (SCP), but also their counterparts in the previous example (Table 2). It should be stressed however that pH control failed in $STR_1$ for about 1 h during exponential growth. This may explain the relatively lower indexes in $STR_1$ Overall, the improvement power of this design was shown to be in the range of 40% and ~60% for $Y_{P/S}$ and $P_v$, respectively. The volumetric productivity of 1.96 kg/m$^3$/h is the highest as ever described before in earlier CalPan fermentation.

**Table 4.** Main performance indexes of a SC-cascade fermentation process using dynamic approach.

| | Control SCP | STR$_1$ | STR$_2$ | STR$_3$ |
|---|---|---|---|---|
| $Y_{X/S}$ (-) | 0.17 | 0.15 | 0.22 | 0.23 |
| $Y_{P/S}$ (-) | 0.18 | 0.15 | 0.22 | 0.25 |
| $P_v$ (kg/m$^3$/h) | 1.24 | 1.33 | 1.72 | 1.96 |

**Claims**

1. Method for producing pantothenate by a semi-continuous cascade fermentation process, using a pantothenate overproducing microorganism, wherein

   a) the process is performed in at least 2 fermentation tanks ($T_1$, $T_2$, to $T_n$), which are interconnected in a row;
   b) medium transfers ($V_T$) are performed for all tanks except for the last tank and the medium transfers are performed from one tank to another in the direction from the first to the last tank;
   c) medium withdrawals ($V_W$) are performed for all tanks, and the medium withdrawals are performed into a separated buffer tank;
   d) feeding profiles can be the same for all tanks $T_n$ or specific for each tank ($T_1$, $T_2$, ...$T_n$)
   e) $F_n$ is the total volume of feeding solution for each tank $T_n$, provided during the whole fermentation process and $F_n$ defines the amounts of medium to be withdrawn and/ or transferred from the tanks $T_n$ as:

   $$F_n \text{ (l)} = (\text{total volume of withdrawn medium from tank } T_n \text{ (l))} + (\text{total volume of transferred medium out of tank } T_n \text{ (l))} - (\text{total volume of transferred medium into tank } T_n \text{ (l))}.$$

2. Method according to claim 1, wherein all the medium transfers comprise to remove the same amount of medium, wherein the amount of medium to be removed each time is 10-20 % of the initial starting volume of one of the fermentation tanks $T_n$.

3. Method according to claims 1 or 2, wherein the medium withdrawals are performed at constant time intervals.

4. Method according to any of the preceding claims, wherein the operation time of the fermentation process in the tanks $T_n$ is more than 60 hours.

5. Method according to any of the preceding claims, wherein medium withdrawals and/or medium transfers are performed more than 2 times for each tank $T_n$ during the fermentation process.

6. Method according to any of the preceding claims, wherein the time interval is less than 10 hours.

7. Method according to any of the preceding claims, wherein the first medium transfer is not performed in the first 6 hours of the fermentation.

8. Method according to any of the preceding claims, wherein the feeding solution comprises a concentration of $PO_4^{3-}$ ions to keep the $PO_4^{3-}$ ion concentration in the fermentation medium of the tanks $T_n$ at a level of 500 mg/l or higher.

9. Method according to any of the preceding claims, wherein the feeding solutions comprises the following ingredients at a minimum concentration of: $KH_2PO_4$, 6 g/l; $K_2HPO_4 \cdot 12H_2O$, 6 g/l; $Na_2HPO_4 \cdot 12H_2O$, 12 g/l.

10. Method according to any of the preceding claims, wherein the dissolved oxygen ($pO_2$) level is kept at $\geq 15\%$

11. Method according to any of the preceding claims, wherein the feeding rate ranges from 11 g/l/h up to 42 g/l/h.

12. Method according to any of the preceding claims, wherein the ratio of the initial starting volume in one of the fermentation tanks $T_n$ / total amount of withdrawn medium from this tank $T_n$ is in the range of 2:1 - 1:2.

13. Method according to any of the preceding claims, wherein the feeding solutions comprises at least 32 g/l glutamate.

14. Method according to any of the preceding claims, wherein at least 2 runs are performed during the whole fermentation process, wherein a run comprises one medium withdrawal and/or transfer out of each tank $T_n$.

15. Method according to any of the preceding claims, wherein the fermentation tanks are provided with glutamate in combination with $NH_4 OH$ and/or $NH_3$ gas.

16. Method according to any of the preceding claims, wherein the feeding rates $f_n$ are the same for all tanks $T_n$.

**Figure 1.**

A

B

**Figure 2.**

**Figure 3.**

**Figure 4.**

**Figure 5.**

**Figure 6**

**Figure 7.**

**Figure 8.**

**Figure 9.**

**Figure 10.**

**Figure 11.**

| | Cascade 1 | | | | | Cascade 2 | | | | | Cascade 3 | | | |
|---|---|---|---|---|---|---|---|---|---|---|---|---|---|---|
| Time (h) | $F_1$ g/h | Total V (L) | $V_{T1}$ (L) | $V_{W1}$ (L) | $V_{R1}$ (L) | $F_2$ g/h | Total V (L) | $V_{T2}$ (L) | $V_{W2}$ (L) | $V_{R2}$ (L) | $F_1$ g/h | Total V (L) | $V_{W3}$ (L) | $V_{R3}$ (L) |
| 0 | | 6.00 | | | 6.00 | | 6.00 | | | 6.00 | | 6.00 | | 6.00 |
| 4 | 80 | 6.00 | | | 6.00 | 70 | 6.00 | | | 6.00 | 70 | 6.00 | | 6.00 |
| 6 | 90 | 6.13 | | | 6.13 | 75 | 6.11 | | | 6.11 | 75 | 6.11 | | 6.11 |
| 12 | 100 | 6.55 | | | 6.55 | 80 | 6.46 | | | 6.46 | 80 | 6.46 | | 6.46 |
| 18 | 100 | 7.02 | | | 7.02 | 85 | 6.84 | | | 6.84 | 85 | 6.84 | | 6.84 |
| 24 | 100 | 7.50 | 0.5 | 0.00 | 7.00 | 90 | 7.24 | | | 7.74 | 90 | 7.24 | 0.00 | 7.24 |
| 30 | 105 | 7.97 | 0.5 | 0.00 | 7.49 | 95 | 7.67 | 0.5 | 0.50 | 7.69 | 95 | 7.67 | 0.50 | 7.69 |
| 36 | 105 | 8.46 | 0.5 | 0.00 | 7.49 | 100 | 8.12 | 0.5 | 0.50 | 7.67 | 100 | 8.12 | 1.00 | 7.67 |
| 42 | 110 | 8.96 | 0.5 | 0.00 | 7.51 | 105 | 8.59 | 0.5 | 0.50 | 7.66 | 105 | 8.59 | 1.00 | 7.66 |
| 48 | 110 | 9.48 | 0.5 | 0.10 | 7.43 | 110 | 9.09 | 0.5 | 0.50 | 7.68 | 110 | 9.09 | 1.00 | 7.68 |
| 54 | 110 | 10.00 | 0.5 | 0.10 | 7.35 | 110 | 9.61 | 0.5 | 0.50 | 7.70 | 110 | 9.61 | 1.00 | 7.70 |
| 60 | 110 | 10.52 | 0.5 | 0.10 | 7.27 | 110 | 10.13 | 0.5 | 0.50 | 7.72 | 110 | 10.13 | 1.00 | 7.72 |
| 66 | 110 | 11.04 | 0.5 | 0.10 | 7.19 | 110 | 10.65 | 0.5 | 0.50 | 7.74 | 110 | 10.65 | 1.00 | 7.74 |
| 72 | 110 | 11.56 | 0.5 | 0.10 | 7.11 | 110 | 11.17 | 0.5 | 0.50 | 7.76 | 110 | 11.17 | 1.00 | 7.76 |

## Figure 12.

**Figure 13.**

**Cascade I**          **Cascade II**          **Cascade III**

**Cascade I**          **Cascade II**          **Cascade III**

European Patent
Office

**EUROPEAN SEARCH REPORT**

Application Number

EP 06 02 2821

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/103274 A (DSM IP ASSETS BV [NL]; EISELE FRANK [CH]) 3 November 2005 (2005-11-03) * page 4, paragraph 3 * * page 7 - page 8; example 1 * | 1,3,4, 10,11,15 | INV. C12P13/02 |
| Y | DATABASE WPI Week 200328 Derwent Publications Ltd., London, GB; AN 2003-286645 XP002433236 & RU 2 193 594 C1 (BIOLOG INSTRUMENTS RES INST) 27 November 2002 (2002-11-27) * abstract * -& RU 2 193 594 C1 (PROIZV RAN M; INST BIOLOG PRIBOROSTROENIJA S) 27 November 2002 (2002-11-27) * abstract * | 1,3 | |
| A | | 2,5-7, 12,14,16 | |
| Y | WO 2005/017172 A (DSM IP ASSETS BV [NL]; BERRY ALAN [US]; LEE CONNIE [FR]; MAYER ANNE FR) 24 February 2005 (2005-02-24) * page 1, line 27 - page 2, line 3 * * page 3, line 34 - page 4, line 29 * * page 5, line 18 - page 6, line 4 * * page 6, line 26 - page 7, line 4 * * page 7, line 27 - line 36 * * page 16, line 18 - page 17, line 16 * * claims 1-6,9,11 * | 1,3,4, 10,11 | TECHNICAL FIELDS SEARCHED (IPC) C12P |
| A | | 2,5-9, 14,16 | |

-/--

The present search report has been drawn up for all claims

| Place of search | Date of completion of the search | Examiner |
|---|---|---|
| Berlin | 21 May 2007 | Schröder, Gunnar |

EPO FORM 1503 03.82 (P04C01)

**European Patent Office**

**EUROPEAN SEARCH REPORT**

Application Number

EP 06 02 2821

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 5 962 286 A (ANASTASSIADIS SAVAS [DE] ET AL) 5 October 1999 (1999-10-05)<br>* column 3, line 58 - column 4, line 21 *<br>* column 17, line 25 - column 18, line 59 *<br>* claims 7,11 * | 1 | |
| A | | 8,9 | |
| Y | ANASTASSIADIS SAVAS ET AL: "Continuous gluconic acid production by the yeast-like Aureobasidium pullulans in a cascading operation of two bioreactors."<br>APPLIED MICROBIOLOGY AND BIOTECHNOLOGY DEC 2006,<br>vol. 73, no. 3, December 2006 (2006-12), pages 541-548, XP002433449<br>ISSN: 0175-7598<br>Published online: 12. August 2006<br>* abstract *<br>* page 542, column 1, line 17 - line 31 *<br>* page 543, column 2, paragraph 2 - page 544, column 2, paragraph 1; figure 1 * | 1 | |
| | | | **TECHNICAL FIELDS SEARCHED (IPC)** |
| Y | WO 2005/028659 A (BASF AG [DE]; BECK CHRISTINE [DE]; BITTERLICH STEFAN [DE]; HARZ HANS-P) 31 March 2005 (2005-03-31)<br>* page 22, paragraph 2 - page 24, paragraph 1 *<br>* page 41 - page 44 * | 15 | |
| A | | 13 | |

The present search report has been drawn up for all claims

| Place of search | Date of completion of the search | Examiner |
|---|---|---|
| Berlin | 21 May 2007 | Schröder, Gunnar |

EPO FORM 1503 03.82 (P04C01)

**ANNEX TO THE EUROPEAN SEARCH REPORT
ON EUROPEAN PATENT APPLICATION NO.**

EP 06 02 2821

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2007

| Patent document cited in search report | | Publication date | Patent family member(s) | | Publication date |
|---|---|---|---|---|---|
| WO 2005103274 | A | 03-11-2005 | KR 20070001244 | A | 03-01-2007 |
| RU 2193594 | C1 | 27-11-2002 | NONE | | |
| WO 2005017172 | A | 24-02-2005 | BR PI0413542 | A | 17-10-2006 |
| | | | WO 2005017159 | A2 | 24-02-2005 |
| | | | CN 1882691 | A | 20-12-2006 |
| | | | JP 2007502102 | T | 08-02-2007 |
| | | | KR 20060064629 | A | 13-06-2006 |
| US 5962286 | A | 05-10-1999 | NONE | | |
| WO 2005028659 | A | 31-03-2005 | DE 10344200 | A1 | 04-05-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0121772 A
- WO 2004005525 A
- US 60262995 B
- WO 02061108 A2
- US 20050124030 A
- WO 02061108 A

### Non-patent literature cited in the description

- **COODIJER et al.** *Enzyme and Microbial Technology,* 1996, vol. 18, 202-219
- **KAZUYUKI et al.** *Chem. Eng. Comm.,* 1987, vol. 52, 61-74
- **KULOZICK et al.** *J. Biotechnol,* 1992, vol. 22, 107-116
- **SCHURE TER et al.** *Microbiol. Rev.,* 2000, vol. 24, 67-83
- **BELITSKY et al.** *J. Bacteriol.,* 1998, vol. 180, 6298-6305
- **BELITSKY et al.** *J. Bacteriol.,* 2004, vol. 186, 3399-3407
- **KUNST et al.** *Nature,* 1997, vol. 390, 249-256
- **FISHER et al.** *Mol. Microbiol.,* 1999, vol. 32, 223-232
- Utilization of amino acids and other nitrogen-containing compounds. **FISHER et al.** Gram-positive Bacteria: Biochemistry, Physiology, and Molecular Biology. American Society for Microbiology Press, 1993, 221-228
- **DESTH ; STÜLKE.** *Microbiol.,* 2003